# EUROPEAN PATENT SPECIFICATION

(11) **EP 4 396 347 B1**
(45) Date of publication and mention of the grant of the patent: **11.02.2026**
(21) Application number: 22773548.7
(22) Date of filing: 30.08.2022
(51) Int. Cl.: C12N 15/113, A61K 31/7088

(54) **CHIMERIC COMPLEX AND THERAPEUTIC USES THEREOF**
CHIMÄRER KOMPLEX UND THERAPEUTISCHE VERWENDUNGEN DAVON
COMPLEXE CHIMÉRIQUE ET SES UTILISATIONS THÉRAPEUTIQUES

(30) Priority: 31.08.2021 IT 202100022610
(43) Date of publication of application: 10.07.2024
(73) Proprietor: Università Degli Studi Di Torino, 10124 Torino (IT)
(72) Inventor: TAVERNA, Daniela, 10023 Chieri (Torino) (IT); QUIRICO, Lorena, 10078 Venaria Reale (Torino) (IT); ORSO, Francesca, 10141 Torino (IT)
(74) Representative: Comoglio, Elena
(86) International application number: PCT/IB2022/058099
(87) International publication number: WO 2023/031778

(56) References cited:
- F. ORSO ET AL: "miR-214 and miR-148b Targeting Inhibits Dissemination of Melanoma and Breast Cancer", CANCER RESEARCH, vol. 76, no. 17, 1 September 2016 (2016-09-01), US, pages 5151 - 5162, XP055692657, ISSN: 0008-5472, DOI: 10.1158/0008-5472.CAN-15-1322
- LORENA QUIRICO ET AL: "Axl-148b chimeric aptamers inhibit breast cancer and melanoma progression", INTERNATIONAL JOURNAL OF BIOLOGICAL SCIENCES, vol. 16, no. 7, 1 January 2020 (2020-01-01), pages 1238 - 1251, XP055691991, ISSN: 1449-2288, DOI: 10.7150/ijbs.39768
- CATUOGNO SILVIA ET AL: "Selective delivery of therapeutic single strand antimiRs by aptamer-based conjugates", JOURNAL OF CONTROLLED RELEASE, ELSEVIER, AMSTERDAM, NL, vol. 210, 19 May 2015 (2015-05-19), pages 147 - 159, XP029217588, ISSN: 0168-3659, DOI: 10.1016/J.JCONREL.2015.05.276
- ESPOSITO CARLA L ET AL: "A combined microRNA-based targeted therapeutic approach to eradicate glioblastoma stem-like cells", JOURNAL OF CONTROLLED RELEASE, ELSEVIER, AMSTERDAM, NL, vol. 238, 21 July 2016 (2016-07-21), pages 43 - 57, XP029712472, ISSN: 0168-3659, DOI: 10.1016/J.JCONREL.2016.07.032
- HUANG LIN ET AL: "Light-Inducible Exosome-Based Vehicle for Endogenous RNA Loading and Delivery to Leukemia Cells", vol. 29, no. 9, 9 January 2019 (2019-01-09), DE, pages 1807189, XP055913313, ISSN: 1616-301X, Retrieved from the Internet <URL:https://api.wiley.com/onlinelibrary/tdm/v1/articles/10.1002%2Fadfm.201807189> DOI: 10.1002/adfm.201807189

## Description

The present invention falls within the field of therapeutic treatments of tumor diseases, particularly solid tumors, more particularly solid tumors expressing the oncogene *axl.*

Tumor diseases are typically characterized by progression through successive, increasingly severe stages. In an initial stage, normal cells, as a result of genetic modifications, begin to proliferate abnormally in a microenvironment consisting of stromal cells embedded in a remodelled extracellular matrix infiltrated by immune cells. Cancer cells that acquire the ability to invade adjacent tissues, intravasate, move through the vascular system, stop in the capillaries and extravasate into the surrounding tissue parenchyma give rise to distant metastases. Since metastatic spread is responsible for over 90% of cancer-related deaths, a great effort in the field of clinical and pharmacological research is aimed at identifying appropriate therapies which allow metastatic development to be stopped or at least slowed down.

In recent years, miRNAs, i.e., small non-coding RNAs acting as negative post-transcriptional regulators for their target genes, have been shown to be involved in tumor biology. In particular, in a vast majority of cases, the formation and progression of tumor disease was found to be associated with aberrant expression of certain miRNAs, and this finding is supported by growing emerging evidence. The first paper on the role of miRNAs in cancer appeared in 2002 and referred to the consequences of miR-15 and miR-16 deletion in chronic lymphocytic leukemia (CLL) (Calin, GA, et al., (2002) "Frequent deletions and down-regulation of micro- RNA genes miR15 and miR16 at 13q14 in chronic lymphocytic leukemia"; Proc Natl Acad Sci 99: 15524-15529). Over 32,500 studies have been published since then. Among the plurality of miRNAs identified so far, let-7 miRNAs, miR-29 family, miR-34 and miR-148b were shown to act as suppressors of "tumors or metastases", whereas the miRNA 17-92 cluster, miR-21, miR-10b and miR-214 were shown to play a role in promoting tumor growth or spread, depending on the tumor context. In particular, recent studies have shown that miR-148b controls breast cancer progression by coordinating a large number of target molecules, including ITGα5 integrin, its downstream actors ROCK1 and PIK3CA/p110α (Cimino, D, et al. (2013) "miR148b is a major coordinator of breast cancer progression in a relapse-associated microRNA signature by targeting ITGA5, ROCK1, PIK3CA, NRAS, and CSF1", FASEB J 27: 1223-1235) and the cell adhesion molecule ALCAM (Penna, E, et al. (2013) "miR-214 coordinates melanoma progression by upregulating ALCAM through TFAP2 and miR-148b downmodulation", Cancer Res 73: 4098-4111). Furthermore, miR-148b expression was shown to be negatively regulated by the pro-metastatic miR-214, thus suggesting that miR-148b acts antagonistically in controlling the spread of breast cancer and melanoma (Orso, F, et al. (2016) "miR-214 and miR-148b Targeting Inhibits Dissemination of Melanoma and Breast Cancer"; Cancer Res 76: 5151-5162).

Due to the role of miRNAs in cancer progression, cancer therapies based on their use have been developed.

Dettori D, Orso F, Penna E, et al. (2018) "Therapeutic Silencing of miR-214 Inhibits Tumor Progression in Multiple Mouse Models". Mol Ther.;26(8):2008-2018 proposes miR-214 as a promising target for anti-metastatic therapies and describes an anti-miR-214 oligonucleotide capable of inhibiting the pro-metastatic functions of miR-214.

International patent application WO2012/049108 discloses a nucleotide aptamer capable of binding to and inhibiting the tyrosine kinase receptor *axl.* As is well known, this receptor is expressed at high levels by several types of tumors, including melanoma and breast tumor, while it is poorly expressed in normal cells.

International patent application WO2021/044282 discloses a chimeric complex formed by the anti*-axl* nucleotide aptamer of WO2012/049108 associated with an miRNA through complementary sticky sequences.

However, in the field of anticancer therapies there is a strong need to constantly develop new therapeutic tools that will increase the effectiveness and/or specificity and/or reduce the side effects of existing therapies.

There is also an ongoing need to provide new therapeutic strategies which are suited for targeting the progression of tumor diseases, thus making it possible to prevent, slow down and/or inhibit the onset of tumor metastases, while hampering the onset of any adverse side effects.

The need to provide alternative therapeutic strategies, which is felt to be of fundamental importance in the field of anticancer therapies, is also caused by the fact that some patients may develop resistance to certain anticancer therapies, such as chemotherapy. This problem is particularly felt in the case of tumors expressing the tyrosine kinase receptor *axl*, whose presence on the surface of tumor cells often leads to the onset of resistance.

In order to meet these and other needs, the present invention provides the chimeric complex as defined in the appended claim 1, and the pharmaceutical composition comprising it.

Additional features and advantages of the invention are defined in the dependent claims, which form an integral part of the description.

As will be apparent from the following detailed description, the present invention provides a chimeric complex which is defined by a combination of features capable of providing an effective antitumor activity, in particular anti-metastatic activity, accompanied by significant selectivity of action.

In the context of the present description, the term "chimeric complex" refers to a macromolecular complex comprising: i) an aptamer directed against the tyrosine kinase receptor *axl* (for the sake of brevity hereinafter referred to as "anti-*axl* aptamer"), wherein the aptamer is an RNA aptamer or a DNA aptamer, and ii) a DNA sponge sequence directed against miR-214 (for the sake of brevity hereinafter referred to as "miR-214sponge"), wherein the above two components are capable of exerting different actions.

The chimeric complex of the invention is sometimes hereinafter referred to as "axl-miR-214sponge".

Generally, the term "aptamer" indicates a single stranded oligonucleotide molecule capable of binding to a certain target molecule, for example a cell transmembrane protein, with high affinity and selectivity. The *axl* receptor, i.e., the target of the anti*-axl* aptamer of the chimeric complex according to the invention, is known to be aberrantly expressed on the surface of a large prevalence of different tumor cells, where it exerts oncogenic activity.

The anti*-axl* aptamer of the chimeric complex according to the invention comprises, from the 5' end to the 3' end:
(i) a single stranded nucleic acid sequence (RNA or DNA) capable of binding to the tyrosine kinase receptor *axl*;
(ii) a linker element consisting of a linear unsubstituted alkyl chain containing 4 to 20 carbon atoms; and
(iii) a first sticky sequence consisting of a single stranded nucleic acid sequence (RNA or DNA) 15 to 20 bases in length.

The linker element is a linear unsubstituted alkyl chain containing 4, 5, 6, 7, 8, 9, 10, 11, 12, 13, 14, 15, 16, 17, 18, 19, or 20 carbon atoms. The linear unsubstituted alkyl chain preferably contains 6 to 18 carbon atoms, more preferably 12 carbon atoms.

In one embodiment of the invention, the single stranded nucleic acid sequence capable of binding to the *axl* aptamer is the "GL21.T" RNA sequence described in WO2012/049108 (SEQ ID NO:1).

In another embodiment of the invention, the first single-stranded sticky nucleic acid sequence of the anti*-axl* aptamer is the RNA sequence of SEQ ID NO:2.

With reference to both of the above-mentioned embodiments, the RNA sequence of SEQ ID NO:1 and/or the RNA sequence of SEQ ID NO:2 can be replaced by the respective modified DNA sequence in which each U (uridine) of SEQ ID NO:1 and/or SEQ ID NO:2 is replaced by a dU (deoxyuridine).

In fact, from WO20124095A1 and from the paper by Amero P. et al. (2021), J. Am. Chem. Soc. 143, 20, 7655-7670 the conversion of an RNA aptamer into a modified DNA aptamer is known to provide prolonged stability and improved antitumor activity.

The chimeric complex according to the invention also includes a second element consisting of an oligonucleotide containing a DNA sponge directed against miR-214 (for the sake of brevity referred to as "miR-214sponge"). This comprises, from the 5' end to the 3' end:
(iv) a binding sequence consisting of a single stranded DNA containing a plurality of miR-214 binding regions interspersed with one or more linking regions, and
(v) a second sticky single stranded DNA sequence having the same base length as the first sticky single stranded nucleic acid sequence identified above in (iii) and complementary thereto.

In one embodiment of invention, the binding sequence identified above in (iv) is SEQ ID NO:3. However, in other embodiments of invention, the binding sequence SEQ ID NO:3 is modified in the nucleotide sequence of the miR-214 binding regions, the number of miR-214 binding regions (e.g., 2, 3 or 4), and/or the length of the linking regions.

Non-limiting examples of modified binding sequences are SEQ ID NOs:7-28 shown below.

In one embodiment of invention, the second sticky sequence of the miR-214sponge identified above in (v) is the sequence SEQ ID NO:4.

As mentioned above, the first and second sticky sequences of the anti*-axl* aptamer and the miR-214sponge, respectively, have the same length in terms of bases and are complementary to each other. As a result, the annealing of these two nucleotide sequences allows the association of the aptamer with the oligonucleotide, thereby the formation of the chimeric complex.

The present invention is based on the results obtained by the inventors in the experimentation and research activities described in the following experimental section.

In short, *in vitro* studies carried out by the present inventors with *axl*-expressing breast cancer and melanoma cells revealed that the use of the chimeric complex according to the invention results in a significant reduction in migration, invasiveness, and transendothelial migration of tumor cells, while increasing the direct targets of the TFAP2γ and ITGα3 miR-214 and decreasing the indirect targets of the ALCAM and ITGα5 miR-214, thus proving to be able to therapeutically affect the axis formed by miR-214 and miR-148b. In fact, miR-148b is a small anti-metastatic RNA that is inhibited by miR-214, which is capable of silencing ALCAM and ITGα5. Furthermore, treatment with the chimeric complex of the invention inhibits the formation and growth of breast cancer cell mammospheres *in vitro.* The effects mentioned above only occur in cells expressing *axl* on their cell surface, not in cells that do not express this receptor. In addition, the inventors found that while the anti*-axl* aptamer alone can also block the metastatic characteristics of tumor cells expressing *axl,* the chimeric complex of the invention has a significantly higher effect than the anti*-axl* aptamer alone, suggesting a combined activity of the anti*-axl* aptamer and the miR-214sponge.

Lastly, when injected into a primary tumor mass or systemically injected into the tail vein in mice, the chimeric complex according to the invention showed a strong ability to block the metastatic process *in vivo.*

In the light of the above, the chimeric complex according to the invention represents an innovative therapeutic tool in the oncological field, which is particularly effective in counteracting (i.e., inhibiting or reducing) tumor invasiveness and metastatic progression, and at the same time characterized by a considerable reduction of adverse side effects thanks to the particular selectivity of action of the aptamer which is capable of mediating the specific binding of said complex to the *axl*-positive target tumor cell and its internalization. In addition, the presence of the miR-214sponge further increases the selectivity of action, making the chimeric complex of the invention particularly effective against solid tumors in which both *axl* and miR-214 are overexpressed, such as melanoma, pancreatic tumor, stomach tumor, prostate tumor, lung tumor, breast tumor (especially triple-negative breast tumor), ovary tumor.

Preferably, the chimeric complex according to the invention is nuclease-resistant.

For this purpose, one or more pyrimidine bases of the chimeric complex according to the invention is/are preferably replaced with the corresponding 2'-fluoropyrimidine (replacement of all pyrimidine bases with the corresponding 2'-fluoropyrimidine is particularly preferred). It is also preferable for one or more purine bases of the chimeric complex to be replaced with the corresponding 2'-O-methylpurine. Replacement of purines with the corresponding 2'-O-methylpurine is particularly preferred in the two sticky sequences.

In one embodiment of the invention, in addition to the nucleotide replacements described above, or alternatively thereto, in order to increase the nuclease-resistance, the 3' end of the first sticky sequence (e.g., SEQ ID NO: 2) and/or the 3' end of the second sticky sequence (e.g., SEQ ID NO: 4) in the chimeric complex of the invention is/are locked by conjugation with a biotin molecule.

Further nucleotide sequence modifications suitable for conferring nuclease resistance include, for example, but are not limited to, the addition of 2'-amino (2'-NH₂) ribose, monothiophosphates or thiophosphates, modifications to the phosphodiester bond (phosphorothioates and methylphosphonates), the use of phosphoramidates, 2'-O-alkyl ribonucleotides, replacement with locked nucleic acids (LNA) or peptide nucleic acids (PNA).

In another embodiment of the invention, the chimeric complex of the invention additionally comprises polyethylene glycol (PEG) or cholesterol in order to decrease renal clearance.

The embodiments of the invention described above can be combined with one another and the various achievable combinations fall within the scope of the invention.

Thanks to its targeted anti-tumor, in particular anti-metastatic activity, the chimeric complex according to the invention is suitable for use in the therapeutic treatment of tumor diseases, preferably tumor diseases characterized by deregulated activity of the tyrosine kinase receptor *axl,* more preferably tumors in which both *axl* and miR-214 are overexpressed.

Tumor diseases include, but are not limited to, melanoma, pancreatic tumor, stomach tumor, prostate tumor, lung tumor, breast tumor (especially triple-negative breast tumor), ovary tumor.

A pharmaceutical composition comprising the chimeric complex of the invention as defined above, in combination with at least one pharmaceutically acceptable carrier, excipient and/or diluent, is also within the scope of the invention.

According to the invention, the pharmaceutical composition is suitable for use in the above therapeutic medical applications relating to the chimeric complex.

The pharmaceutical composition of the present invention can be formulated into any suitable dosage form, for example, for subcutaneous, intravenous, intra-arterial, intraperitoneal, intramuscular, intranasal, or inhalatory administration.

In an alternative embodiment, the pharmaceutical composition according to the invention can be formulated into a dosage form suitable for local intra-tumor administration, for example by injection under computed tomography guidance.

Of course, the selection of suitable carriers, excipients and/or diluents is carried out depending on the desired form of administration and this selection is within the skills of those of ordinary skill in the art. The selection of the active principle dose and dosage regimen also falls within the skills of those of ordinary skill in the art, and the selection thereof depends on several factors, such as for example the age and weight of the patient, the type of tumor, the degree of progression of the disease, as well as the size of the tumor mass to be treated.

The invention is further described in the examples below, with reference to the accompanying drawings, wherein:

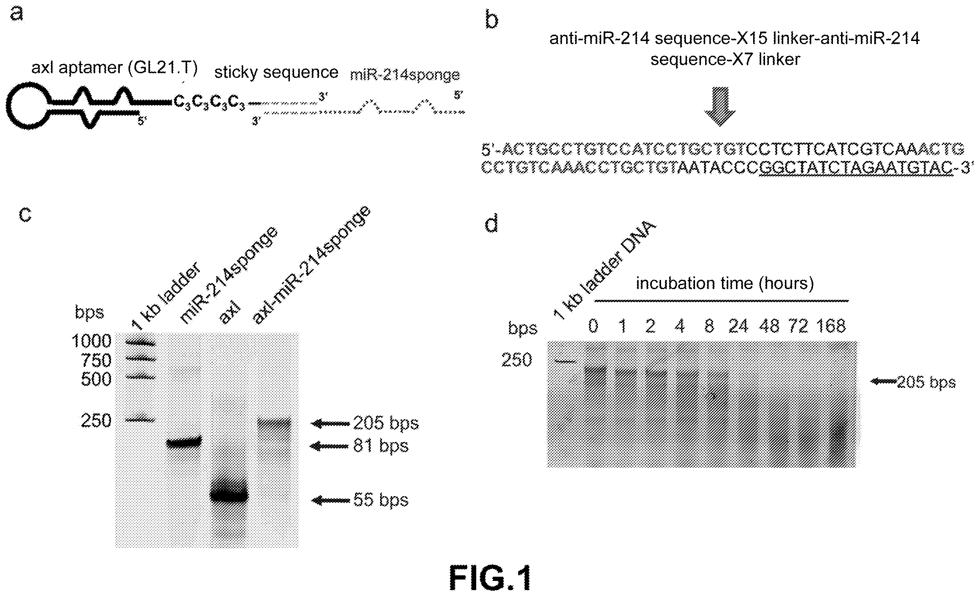

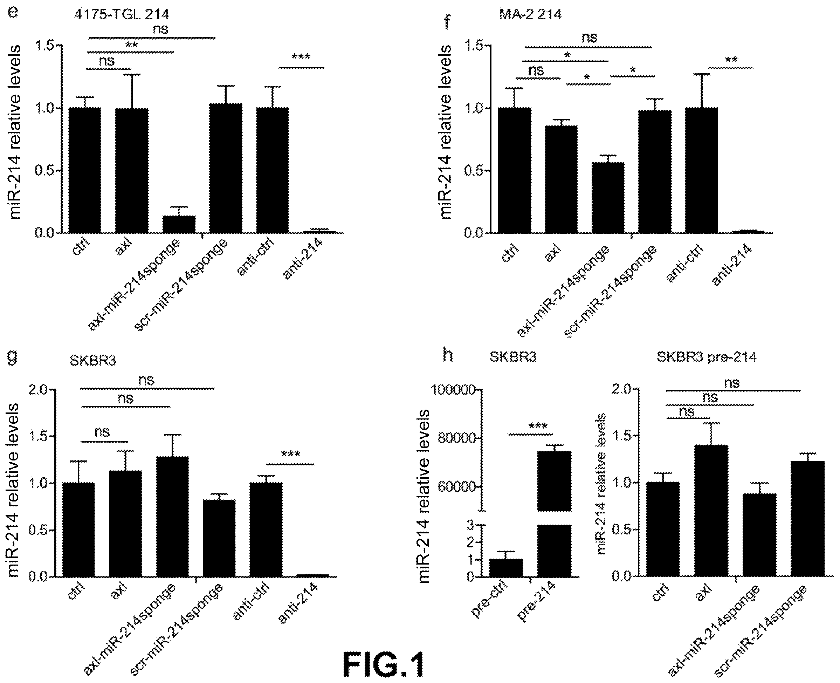

Figure 1 shows the structure of the chimeric complex of the invention and its impact on miR-214 expression in *axl*-positive or *axl*-negative tumor cells. (a) A schematic representation of the chimeric complex according to the invention ("axl-miR-214sponge"). (b) miR-214sponge sequence. (c) An image depicting a polyacrylamide gel electrophoresis to check the correct formation of the chimeric complex. The first band corresponds to miR-214sponge, the second band to the anti*-axl* aptamer, and the third band to the chimeric axl-miR-214sponge complex according to the invention. (d) Images depicting non-denaturing polyacrylamide gel electrophoresis experiments showing the stability of the axl-miR-214sponge chimeric complex (4 µM) in 80% human serum at the indicated time points (h=hours). For (c-d), reference = 1kb. (e-h) miR-214 relative expression levels obtained by qRT-PCR in 4175-TGL and MA-2 cells expressing *axl* and overexpressing miR-214, or in *axl*-negative SKBR3 cells, after treatment with the control solution (ctrl), the anti-*axl* aptamer alone, the axl-miR-214sponge of the invention, or the scr-miR-214sponge. Transfection with anti-miR-214 (anti-214) or pre-miR-214 (pre-214) or their respective controls (anti-ctrl or pre-ctrl) was also performed and the respective expression levels of miR-214 were analyzed. Results are shown as fold changes (mean±SD) versus controls (ctrl or anti-ctrl), normalized to the small nucleolar RNA U44 levels. Three independent experiments were performed in triplicate and a representative one is shown. Ns = not significant, *p <0.05, ** p<0.01, ***p <0.001. SD = Standard Deviation.

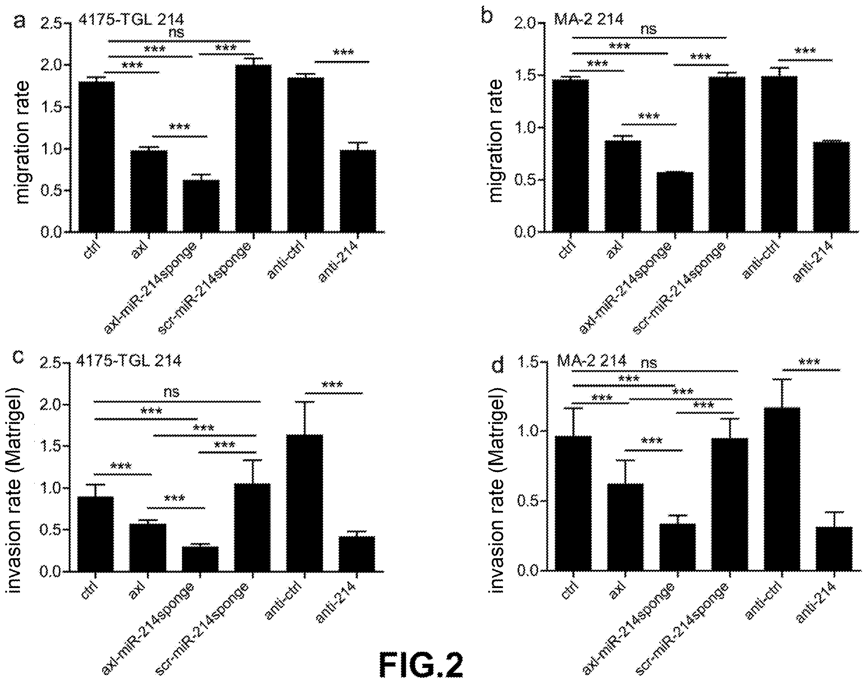

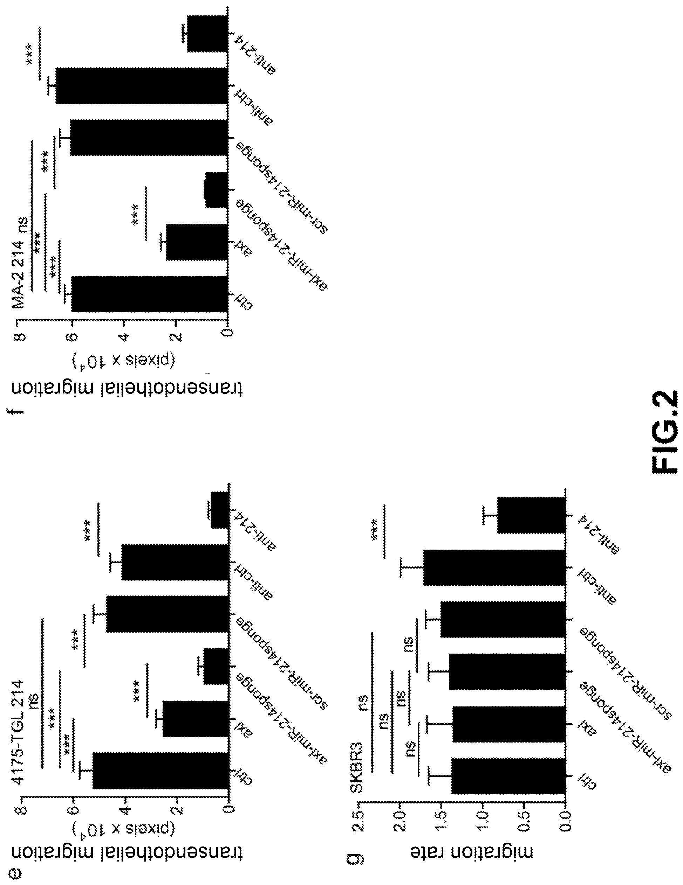

Figure 2 shows that the chimeric complex of the invention is capable of inhibiting breast tumor and melanoma cell motility. (a-g) Transwell migration (a-b, g), invasiveness through Matrigel (c-d) or transendothelial migration through a HUVEC monolayer (e-f) assays for 4175-TGL and MA-2 cells expressing *axl* and overexpressing miR-214, and for *axl*-negative SKBR3 cells, treated with the ctrl solution, the anti*-axl* aptamer alone, or the axl-miR-214sponge or scr-miR-214sponge complex. Transfection with anti-miR-214 (anti-214) and its control (anti-ctrl) was also performed. The results in the graphs are expressed as the ratio of the mean ± SEM of the area covered by the migrated/invasive cancer cells vs plated cells (a-d, g) or as the mean ± SEM of the area (pixels) covered by the transmigrated cells (e-f). At least two independent experiments (in triplicate) were carried out, and representative results thereof are shown. ns = not significant; *p <0.05, **p <0.01, ***p <0.001; SEM = Standard Error of the Mean.

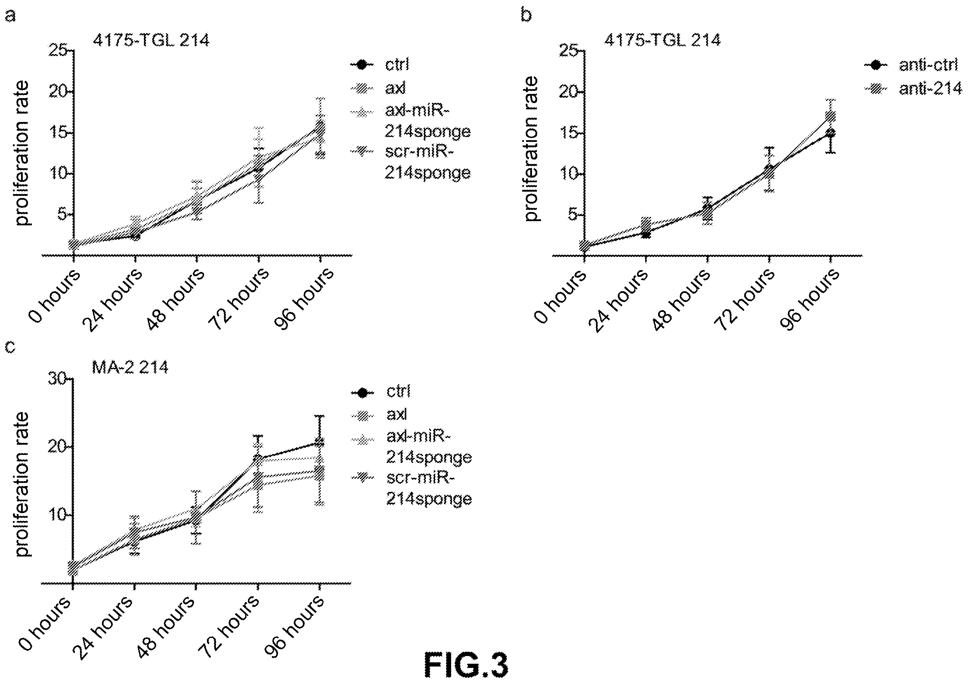

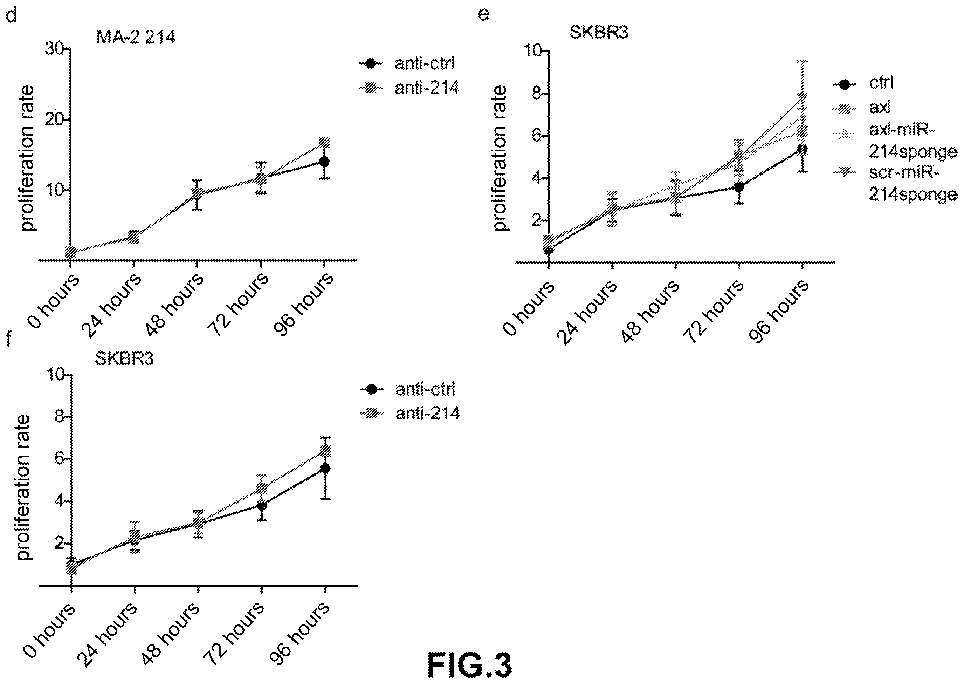

Figure 3 shows that the chimeric complex of the invention has no effects on tumor cell growth. (a-f) Growth assays for 4175-TGL and MA-2 cells expressing *axl* and overexpressing miR-214, and for *axl*-negative SKBR3 cells, treated with the ctrl solution, the anti*-axl* aptamer alone, or the axl-miR-214sponge or scr-miR-214sponge complex. Results are shown as the mean±SD of the ratio between growth and number of plated cells, measured as the optical density at 0-96 hours (h). At least 2 independent experiments were performed in triplicate and representative results thereof are shown. SD = Standard Deviation.

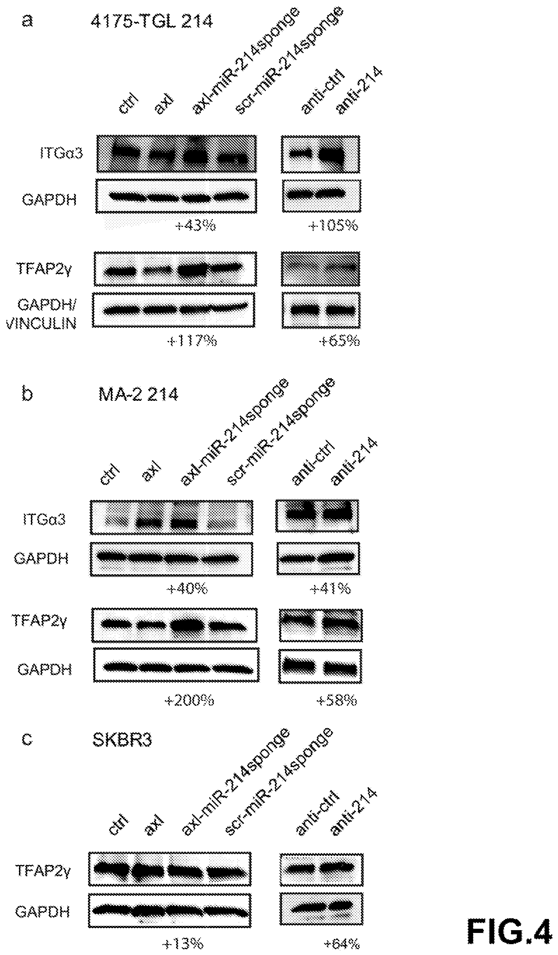

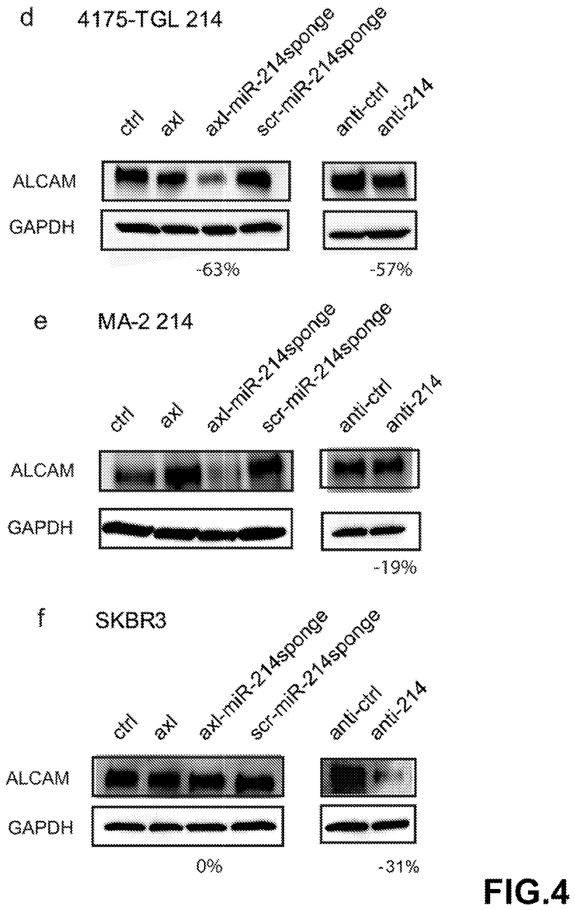

Figure 4 shows that the chimeric complex of the invention modulates the expression of miR-214 targets in *axl*-expressing breast tumor and melanoma cells. (a-f) Western blot analysis of the direct miR-214 targets ITGα3 and TFAP2γ or of the indirect target ALCAM in 4175-TGL and MA-2 cells expressing *axl* and overexpressing miR-214 (a-b, d-e), or in *axl-*negative SKBR3 cells, treated with the control solution, the anti*-axl* aptamer alone, or the axl-miR-214sponge or scr-miR-214sponge complex. Transfection with anti-miR-214 (anti-214) and its control (anti-ctrl) was also performed. Proteins were collected after 48 hours. Protein variations were calculated with reference to the anti-axl or anti-ctrl aptamer, normalized to loading controls (Vinculin or GAPDH), and expressed as percentages (%). At least three independent experiments were performed, and representative results thereof are shown. For the top and left panels, a and d, GAPDH is the same because the blots were incubated with anti-ITGα3 antibody, striped, and incubated with anti-ALCAM antibody.

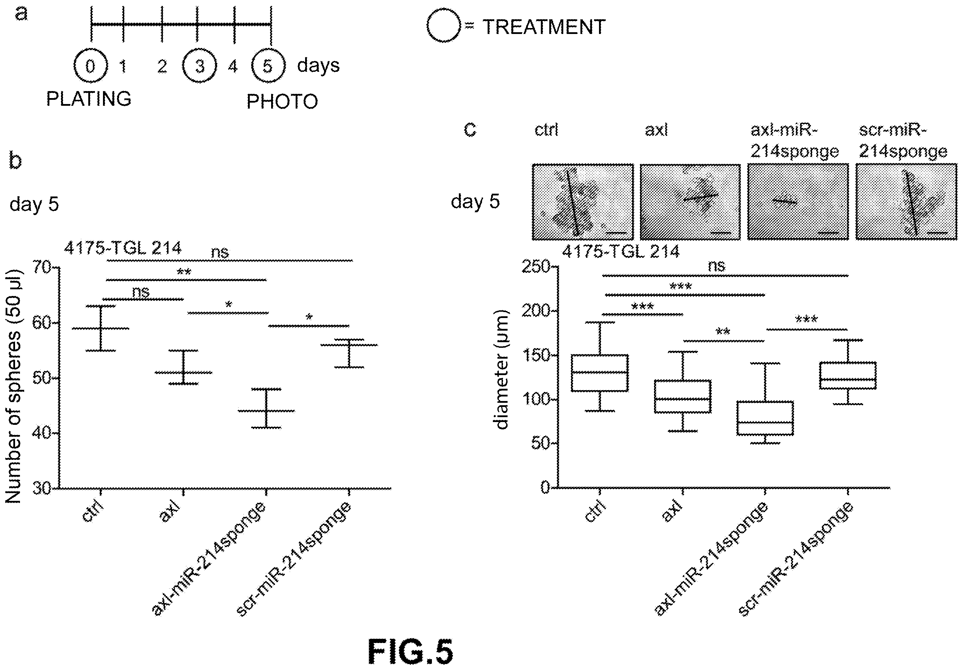

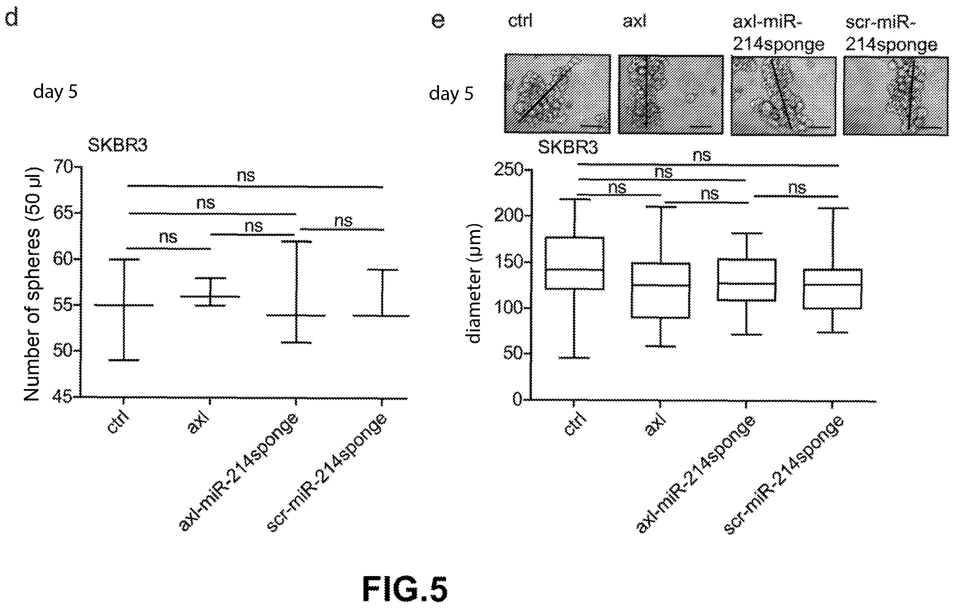

Figure 5 shows that the chimeric complex of the invention reduces the quantity and size of mammospheres derived from *axl*-positive breast tumor cells, but not from *axl*-negative breast tumor cells. (a) Schematic representation of the protocol: 4175-TGL breast tumor cells expressing axl and overexpressing miR-214 or SKBR3 *axl*-negative breast tumor cells were plated (Day 0), maintained in suspension and treated with the ctrl solution, the anti-axl aptamer alone, or the axl-miR-214sponge or scr-miR-214sponge complex. Treatments were repeated on days 3 and 5. (b-d) On day 5, the size and number of spheres were assessed under a microscope. Both the number and size of the spheres are shown as the mean±SEM of the length of the spheres (µm). (b,d) Plots depicting the number of 4175-TGL 214 or SKBR3-derived mammospheres in a volume of 50 µl of medium on day 5, shown as the mean ± SEM. (c-e) Top: Photographs representing mammospheres. Bottom: plots relating to the size of the spheres, shown as the mean ± SEM of the length of the spheres (µm); the black lines correspond to size measurements. At least two independent experiments (in triplicate) were carried out, and representative results thereof are shown. ns = not significant; *p <0.05, **p <0.01, ***p <0.001; SEM = Standard Error of the Mean; Scale bar = 25 µm (c-e).

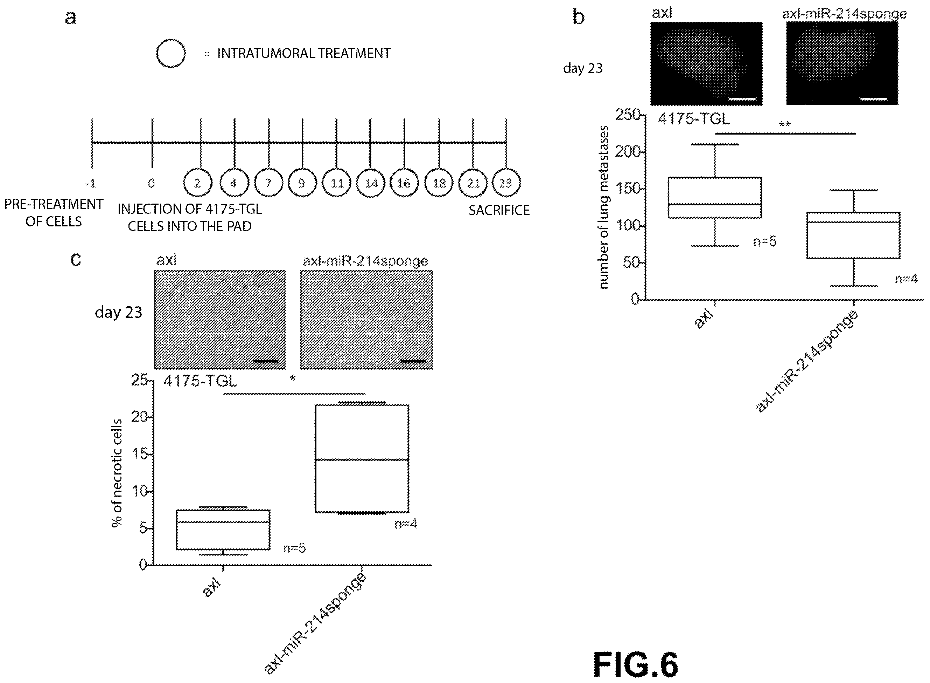

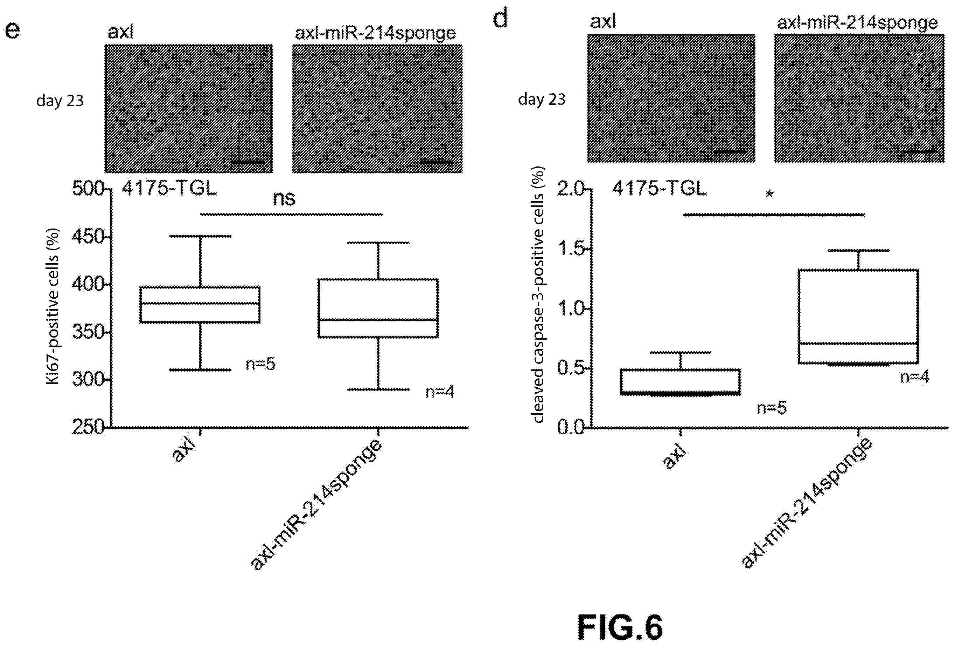

Figure 6 shows that axl-miR-214sponge reduces metastasis and promotes necrosis and apoptosis in breast tumor xenografts. (a) Diagram of the experiment: 4175-TGL breast tumor cells were pretreated for 24 hours with either the anti*-axl* aptamer or the axl-miR-214sponge and then injected orthotopically into the mammary gland of NOD/SCID/IL2R_null mice (NSG). axl or axl-miR-214sponge was administered into the tumors starting on day 2 post-injection (3 treatments/week, 400 pmoles in 100 µl, 10 injections in total, as indicated), and the primary tumors were analyzed. (b) Images depicting the lungs are shown. The plot indicates the number of lung metastases, and the results are reported as the mean±SEM for the indicated number (n) of mice. (c) Primary tumor sections were stained with H&E and the necrotic areas were assessed: representative images are shown above the plots indicating the percentage (%) of the necrotic area vs the total area, shown as the mean±SEM for the indicated number (n) of mice. (d-e) Primary tumors were IHC stained with either a cleaved anti-Caspase-3 antibody (d) or KI67 (e), and the nuclei were counter-stained with hematoxylin (blue): representative images are shown above the plots indicating the percentage (%) of positive cells vs the total number of cells, shown as the mean±SEM for the indicated number (n) of mice (10 fields per mouse were assessed). IHC = immunohistochemistry. H&E = Hematoxylin & Eosin. Scale bar = 100 µm (c) or 25 µm (d, e). ns = not significant; * p< 0.05; ** p< 0.01; *** p <0.001. SEM = Standard Error of the Mean.

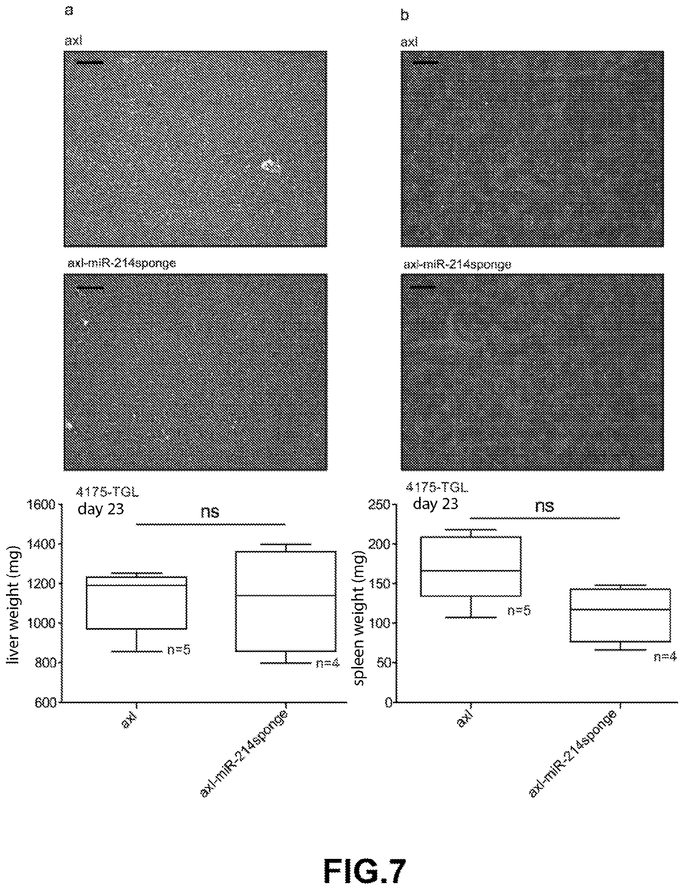

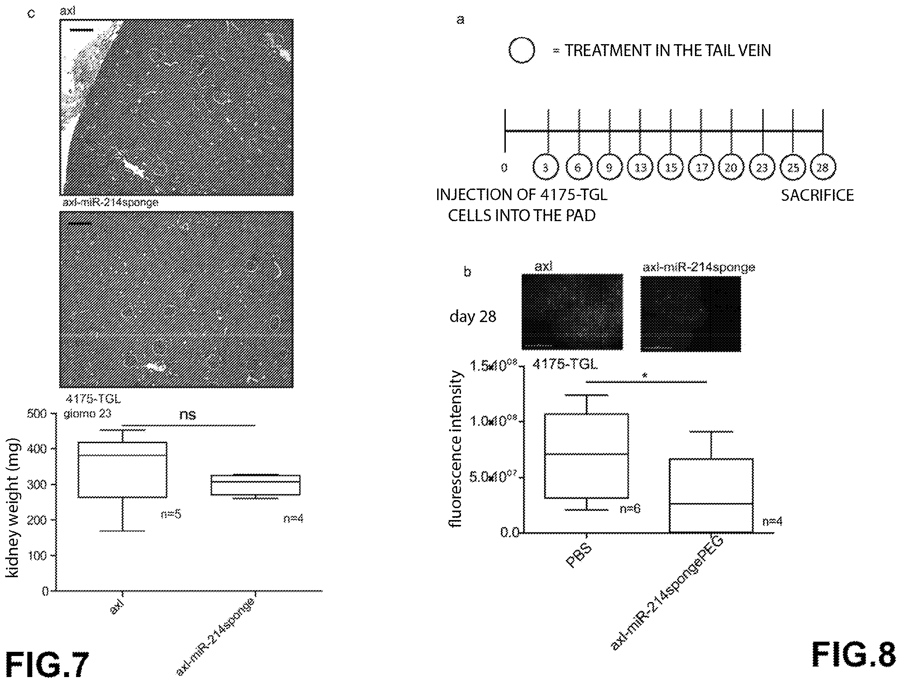

Figure 7 shows that axl-miR-214sponge is not toxic to mice. (a-c) Mice were injected with 4175-TGL cells and treated as described in Figure 6. The liver (a), spleen (b) and kidneys (c) were weighed on day 23 (the day the experiment ended) and the sections were stained with H&E. Photographs depicting the organs (scale bar =100 or 200 µm) are shown above the plots representing the mean±SEM of the weight of the organs for the indicated number (n) of mice. ns = not significant. SEM= Standard Error of the Mean; H&E = Hematoxylin & Eosin; mg = milligrams.
Figure 8 shows that axl-miR-214sponge PEG prevents breast tumor spread in mice injected systemically. (a) Diagram of the experiment. 4175-TGL breast tumor cells were injected orthotopically into the mammary gland of NOD/SCID/IL2R_null mice (NSG), and PBS or the aptamer axl-miR-214sponge PEG was administered into the mouse tail vein starting on day 3 post-injection (3 treatments/week, 1600 pmoles in 100 µl, 10 injections in total, as indicated). (b) Images depicting the lungs and their metastases are shown. The plot indicates the fluorescence of lung metastases, and the results are reported as the mean±SEM for the indicated number (n) of mice. Scale bar = 800 µm. ns = not significant; * p< 0.05; ** p< 0.01; *** p <0.001. SEM = Standard Error of the Mean.

### EXAMPLES

### Materials and Methods

### Cell cultures

MA-2 cells were kindly provided by L. Xu and R.O. Hynes (Xu, L, et al. (2008). Gene expression changes in an animal melanoma model correlate with aggressiveness of human melanoma metastases. Mol Cancer Res 6: 760-769.) and maintained as described in (Penna, E, Orso, F et al. (2011). "microRNA-214 contributes to melanoma tumour progression through suppression of TFAP2C". EMBO J 30: 1990-2007; Penna, E, et al. (2013) "miR-214 coordinates melanoma progression by upregulating ALCAM through TFAP2 and miR-148b downmodulation", Cancer Res 73: 4098-4111). SKBR3 cells were purchased from the American Type Culture Collection (ATCC), while 4175-TGL cells were kindly provided by J. Massagué (Minn, AJ et al. (2005). Genes that mediate breast cancer metastasis to lung. Nature 436: 518-524) and maintained under standard culture conditions. HUVEC cells (human endothelial cells obtained from the umbilical cord) were kindly provided by M.F. Brizzi and maintained as described in (Penna, E, Orso, F et al. (2011). "microRNA-214 contributes to melanoma tumour progression through suppression of TFAP2C". EMBO J 30: 1990-2007; Penna, E, et al. (2013) "miR-214 coordinates melanoma progression by upregulating ALCAM through TFAP2 and miR-148b downmodulation", Cancer Res 73: 4098-4111).

### Reagents and antibodies.

The following were used as anti-miR inhibitors: Anti-miR miRNA Inhibitor Negative Control #1, Anti-miR miRNA Inhibitor hsa-miR-214 (AM12124) (Applied Biosystems). The following reagents were used for the analysis of miRNA expression levels: MicroRNA TaqMan^{®}: Hsa-miR-214 ID 002306, U6 snRNA ID001973, U44 snRNA ID001904 (Applied Biosystems). Primary antibodies: anti-Cleaved Caspase-3 (Asp175) # 9661 (Cell Signaling Technology), anti-KI67 ab15580 (Abcam), anti-ITGα5pAb RM10 kindly provided by G. Tarone (Molecular Biotechnology Center, University of Turin), anti-ITGα3 kindly provided by E. Turco (Molecular Biotechnology Center, University of Turin), anti-CD166/ALCAM mAb MOG / 07 (Novocastra Laboratories), anti-TFAP2γ (6E4/4), anti-GAPDH pAb V-18, anti-ACTIN I-19 pAb (from Santa Cruz Biotechnology), anti-α-tubulin mAb B5-1-2 (Sigma). Secondary antibodies: HRP-conjugated goat anti-mouse IgG, goat anti-rabbit IgG (Santa Cruz Biotechnology).

### Transient transfections and production of stable cell lines.

In order to obtain miRNA transient expression and stable cell lines for the expression of the miRNAs, the cells were treated as described in (Penna, E, Orso, F et al. (2011). "microRNA-214 contributes to melanoma tumour progression through suppression of TFAP2C". EMBO J 30: 1990-2007; Orso, F, et al. (2016) "miR-214 and miR-148b Targeting Inhibits Dissemination of Melanoma and Breast Cancer"; Cancer Res 76: 5151-5162)5).

### Preparation of the chimeric complex.

In order to generate an axl-miR-214sponge chimeric complex falling within the scope of the invention, an miR-214 sponge was conjugated with an anti-*axl* aptamer ("GL21.T") (Cerchia et al. (2012). Targeting Axl with a high-affinity inhibitory aptamer. Mol Ther 20: 2291-2303), by annealing the sequences through their sticky ends.

The following sequences were used:
5'AUGAUCAAUCGCCUCAAUUCGACAGGAGGCUCAC 3' (GL21.T binding sequence capable of binding to the tyrosine kinase receptor axl, SEQ ID NO: 1);
5'GUACAUUCUAGAUAGCC 3' (first sticky sequence, SEQ ID NO:2);
5'ACTGCCTGTCCATCCTGCTGTCCTCTTCATCGTCAAACTGCCTGTCAAACCTG CTGTAATACCC 3' (binding sequence with multiple anti-miR-214 sites, SEQ ID NO:3);
5'GGCTATCTAGAATGTAC 3' (second sticky sequence, SEQ ID NO:4);
5'GGCGCUAGAACCUUCUAAGCGAAUACAUUACCGC 3' (GL21.T scramble, SEQ ID NO:5);

***GL21.T-sticky (anti-axl aptamer):*** 
5'AUGAUCAAUCGCCUCAAUUCGACAGGAGGCUCACXXXXGUACAUUCUAGA
UAGCC 3' (SEQ ID NO:1-XXXX-SEQ ID NO:2);
***GL21.T scramble (scr aptamer):*** 
5'GGCGCUAGAACCUUCUAAGCGAAUACAUUACCGCXXXXGUACAUUCUAGA
UAGCC 3' (SEQ ID NO:5-XXXX-SEQ ID NO:2);
***miR-214sponge:***

5'ACTGCCTGTCCATCCTGCTGTCCTCTTCATCGTCAAACTGCCTGTCAAACCTG
CTGTAATACCCGGCTATCTAGAATGTAC 3' (SEQ ID NO:6).

In the anti-axl and scramble aptamer sequences "XXXX" represents the linear unsubstituted alkyl sequence having 4 to 2 carbon atoms acting as a linker.

### Alternative sequences to SEQ ID NO:3:

The following binding sequences may be used as alternatives to the binding sequence SEQ ID NO:3:

5'ACTGCCTGTCTAACCTGCTGTAACTACCAATACCAAACTGCCTGTCAACCCTG
CTGTCTTTTGT 3' (SEQ ID NO:7)

5’ACTGCCTGTCCGTCCTGCTGTCCCATCTATTTCCTTACTGCCTGTCCACCCTGC
TGTTTAATCC 3’ (SEQ ID NO:8)

5'ACTGCCTGTCCCTCCTGCTGTAACAACCTTCACCGAACTGCCTGTCCATCCTG
CTGTATCATCC 3' (SEQ ID NO:9)

5’ACTGCCTGTCAATCCTGCTGTCACTAATTTCATCCAACTGCCTGTCACCCCTG
CTGTCTATCCC 3’ (SEQ ID NO:10)

5'ACTGCCTGTCCGTCCTGCTGTATCTTACTTCATCAAACTGCCTGTCCCTCCTG
CTGTCCCCCCC 3' (SEQ ID NO:11)

5'ACTGCCTGTCCGTCCTGCTGTCGTATCCTACCTTAAACTGCCTGTCCCACCTG
CTGTATCCCCC 3' (SEQ ID NO:12)

5'ACTGCCTGTCCATCCTGCTGTCATTACTCCCCTTATACTGCCTGTCAATCCTG
CTGTTATTCCC 3' (SEQ ID NO:13)

5'ACTGCCTGTCCATCCTGCTGTCTCCCGTTTCTTTTTACTGCCTGTCCCTCCTGC
TGTCGTCTCC 3' (SEQ ID NO:14)

5'ACTGCCTGTCAATCCTGCTGTATCCTTATCGAATTAACTGCCTGTCCCTCCTG
CTGTCGTCCCC 3' (SEQ ID NO:15)

5'ACTGCCTGTCACCCCTGCTGTTTTCCCATCCCCATTACTGCCTGTCCCACCTG
CTGTCTCTTTT 3' (SEQ ID NO:16)

5'ACTGCCTGTCTATCCTGCTGTTAAAAACCTTTAAATACTGCCTGTCTCCCCTG
CTGTTACCATT 3' (SEQ ID NO:17)

5’ACTGCCTGTCTACCCTGCTGTCCTAAAAATTTATAAACTGCCTGTCCAACCTG
CTGTCCCCTTT 3’ (SEQ ID NO:18)

5’ACTGCCTGTCAACCCTGCTGTAATCTGTGTTTATGTACTGCCTGTCCCCCCTG
CTGTCTCCCCT 3’ (SEQ ID NO:19)

5'ACTGCCTGTCCATCCTGCTGTTTTCCTTTCTCATATACTGCCTGTCAATCCTGC
TGTAATCCGT 3' (SEQ ID NO:20)

5'ACTGCCTGTCTATCCTGCTGTCGTCAAACTTATCATACTGCCTGTCCCTCCTG
CTGTCTACCCT 3' (SEQ ID NO:21)

5'ACTGCCTGTCAACCCTGCTGTGCTCATCCCTCATCAACTGCCTGTCCCTCCTG
CTGTCTCCAAT 3' (SEQ ID NO:22)

5'ACTGCCTGTCACCCCTGCTGTATTTTTCCAATCCGAACTGCCTGTCACCCCTG
CTGTCTCTTAT 3' (SEQ ID NO:23)

5'ACTGCCTGTCCATCCTGCTGTGTCTTAACTCCCATAACTGCCTGTCCATCCTG
CTGTAACCTTT 3' (SEQ ID NO:24)

5’ACTGCCTGTCAAACCTGCTGTCCACCCACTAAACCTACTGCCTGTCCATCCTG
CTGTAATAATT 3’ (SEQ ID NO:25)

5'ACTGCCTGTCAATCCTGCTGTACTCAAATAAATAATACTGCCTGTCAATCCTG
CTGTTCCTCGT 3' (SEQ ID NO:26)

5’ACTGCCTGTCAATCCTGCTGTTTTACCCTCACAATTACTGCCTGTCAACCCTG
CTGTCAATAAT 3’ (SEQ ID NO:27)

5’ACTGCCTGTCAACCCTGCTGTACCTCTCACCTATCAACTGCCTGTCCCCCCTG
CTGTCTACCGT 3’ (SEQ ID NO:28).

All RNAs have modified 2'-F pyrimidines (2'F-Py) to improve stability and have been synthesized at the Synthetic and Biopolymer Chemistry Core, Beckman Research Institute, City of Hope, Duarte, CA. The sticky ends, consisting of 2'-F-Py and 2'-0-Methylpurine (2'OMe-Pu), are underlined. XXXX indicates the linear unsubstituted C4-C20 alkyl sequence acting as a linker. Alternatively, the sequence of the anti*-axl* aptamer may have a PEG at the 5'. To prepare the axl-miR-214sponge, axl-miR-214spongePEG and scramble-miR-214sponge complexes: 1) miR-214sponge sequence was incubated at 95°C for 10 minutes; 2) the aptamer with the sticky ends or scramble sequences were folded (5 min 85°C, 3 min on ice, 10 min at 37°C); 3) equal amounts of aptamer/scramble and miR-214sponge were annealed by incubating them together at 37°C for 30 minutes. Annealing efficiency was checked as described in (Catuogno et al. (2015). Selective delivery of therapeutic single strand antimiRs by aptamer-based conjugates. J Control Release 210: 147-159). In order to treat the cells with the axl, axl-miR-214sponge or scramble-miR-214sponge aptamers, the cells were plated in 24-well plates at 80% confluence and treated 24 hours later with the folded aptamers by adding them to their culture medium.

### Protein or RNA isolation, Western Blot, qRT-PCR

Total protein or RNA extracts, and the Western Blot (WB) and qRT-PCR assays were performed as described in Penna, E, Orso, F et al. (2011). "microRNA-214 contributes to melanoma tumour progression through suppression of TFAP2C". EMBO J 30: 1990-2007.

### Proliferation, migration, invasiveness and transendothelial migration assays

*In vitro* proliferation, migration, invasiveness and transendothelial migration assays were performed as described in (Penna, E, Orso, F et al. (2011). "microRNA-214 contributes to melanoma tumour progression through suppression of TFAP2C". EMBO J 30: 1990-2007, Penna, E, et al. (2013) "miR-214 coordinates melanoma progression by upregulating ALCAM through TFAP2 and miR-148b downmodulation", Cancer Res 73: 4098-4111, Cerchia et al. (2012). Targeting Axl with a high-affinity inhibitory aptamer. Mol Ther 20: 2291-2303).

### Mammosphere formation assays

Mammosphere formation assays were performed as described in the Technical Bulletin of Stem Cell Technologies Tumorsphere, DOCUMENT # 29936 VERSION 1.1.0 JAN 2014 "Culture of Human Breast Cancer Cell Lines", (https://www.stemcell.com/tumorsphere-culture-human-breast-cancer-cell-lines-lp.html) in 24-well plates coated with poly-HEMA (poly-2-hydroxyethylmethacrylate) using two different protocols. In one case, single breast tumor cells (8x10³ cells/well for the 4175-TGL cells, 1x10⁴ cells/well for the SKBR3 cells) were plated (day 0), maintained in suspension in MammoCult Medium (StemCell Technologies) and left untreated (controls = ctrl) or treated with 400 nmol/L of the anti-*axl* miR-214sponge or scr-miR-214sponge complex. Treatments were repeated on days 3 and 5 (200 nmol/L). On day 5, the size and number of the spheres were assessed by using a Zeiss AxioObserver microscope (Zeiss) and the ImageJ software (http://rsbweb.nih.gov/ij/).

For assessing the size, the long side (length) of the spheres was measured. For assessing the number, the total number of spheres was counted in 50 µl volume for each treatment.

### Histology and immunohistochemistry

5 µm thick tissue sections were cut from formalin-fixed, paraffin-embedded (FFPE) tumor specimens and stained with Hematoxylin and Eosin (H&E) for standard histological observations. Immunohistochemical staining (IHC) was performed by using anti-KI67 or anti-cleaved caspase 3 antibodies, with avidin-biotin-peroxidase techniques (Anti-Mouse HRP-DAB Cell & Tissue Staining Kit, R & D Systems). The slides were counterstained with hematoxylin.

### Stability of the axl-miR-214sponge chimeric complex in human serum

The stability of the chimeric complex in human serum was assessed as described in (Catuogno et al. (2015). Selective delivery of therapeutic single strand antimiRs by aptamer-based conjugates. J Control Release 210: 147-159).

### In vivo tumor growth and metastasis assays

All the experiments performed with animals were performed in compliance with ethics. NOD/SCID/IL2R_null (NSG) mice were injected with tumor cells as described in (Penna, E, et al. (2013) "miR-214 coordinates melanoma progression by upregulating ALCAM through TFAP2 and miR-148b downmodulation", Cancer Res 73: 4098-4111, Orso, F, et al. (2016) "miR-214 and miR-148b Targeting Inhibits Dissemination of Melanoma and Breast Cancer"; Cancer Res 76: 5151-5162). Tumor cells were pre-treated 24 hours prior to injection with either the anti-axl aptamer or the axl-miR-214sponge aptamer. As soon as the tumors were palpable, they were treated with axl or miR-214sponge (400 pmol/injection, three injections per week). The mice were sacrificed and analyzed 23 days after the injections with the 4175-TGL cells. Alternatively, the mice were injected with 4175-TGL cells, and the animals were treated with an injection into the tail vein from Day 3 with PBS or axl-miR-214spongePEG (1600 pmol/injection, three injections per week) and sacrificed on Day 28. In both cases, the weight and morphology of the primary tumor and the lung or liver metastases were assessed as described in (Penna, E, et al. (2013) "miR-214 coordinates melanoma progression by upregulating ALCAM through TFAP2 and miR-148b downmodulation", Cancer Res 73: 4098-4111, Orso, F, et al. (2016) "miR-214 and miR-148b Targeting Inhibits Dissemination of Melanoma and Breast Cancer"; Cancer Res 76: 5151-5162). Organ size (liver, spleen, kidney) (weight) and morphology (H & E) were analyzed at the end point.

### Statistical analysis

All results are presented as the mean ± Standard Deviation (SD) or ± Standard Error of the Mean (SEM), as indicated, and the two-tailed Student's t-test was used for comparisons. * = p <0.05; ** = p <0.01; *** = p <0.001 were considered statistically significant. ns = indicates a non-statistically significant p-value.

### Results

### The axl-miR-214sponge chimeric complex and its impact on miR-214 expression

The therapeutic potential resulting from the modulation of miR-214 levels had previously been demonstrated (Orso, F, et al. (2016) "miR-214 and miR-148b Targeting Inhibits Dissemination of Melanoma and Breast Cancer"; Cancer Res 76: 5151-5162)., Dettori D, Orso F, Penna E, et al. (2018) "Therapeutic Silencing of miR-214 Inhibits Tumor Progression in Multiple Mouse Models". Mol Ther.;26(8):2008-2018). With the aim of delivering a sponge, i.e., a sequence designed to inhibit miR-214, specifically to breast tumor or melanoma cells, the inventors generated the chimeric axl-miR-214sponge complex (Figure 1a). In detail, a well-known RNA aptamer, GL21.T, was used, which is able to bind with great affinity and specificity to *axl* (Cerchia et al. (2012). Targeting Axl with a high-affinity inhibitory aptamer. Mol Ther 20: 2291-2303), i.e., an oncogenic receptor overexpressed on the surface of tumor cells and particularly on triple-negative breast tumor and melanoma cells (Quirico et al. (2020) "Axl-148b chimeric aptamers inhibit breast cancer and melanoma progression" IJBS 16:1238-1251). To reduce steric hindrance and preserve proper folding of the complex, the original sponge DNA sequence consisting of 8 miR-214 binding sites, each 21 nucleotides in length, interspersed with 15 spacer nucleotides, perfectly complementary to the miR-214 seed region and with a mismatch in position 9-12 (Penna, E, Orso, F et al. (2011). "microRNA-214 contributes to melanoma tumour progression through suppression of TFAP2C". EMBO J 30: 1990-2007, Orso, F, et al. (2016) "miR-214 and miR-148b Targeting Inhibits Dissemination of Melanoma and Breast Cancer"; Cancer Res 76: 5151-5162).), was shortened. In the new sequence there are two miR-214 binding sites separated by 15 nucleotides which act to give flexibility to the sequence and improve miR-214 binding (Figure 1b). In addition, the terminal part of the miR-214 sponge contains 7 nucleotides and a sticky sequence that binds to the aptamer to obtain the chimeric complex.

The folding efficiency of the molecule was analyzed for each preparation by using a non-denaturing polyacrylamide gel: as shown, the chimera corresponds to the band at the top, followed by bands related to the miR-214 sponge and the anti*-axl* aptamer, indicating proper formation of the axl-miR-214sponge (Figure 1c). In parallel, to assess *in vitro* serum stability, we incubated the chimeric complex with human serum at different time points starting from time zero (0 hours) up to 168 hours (Figure 1d). After analyzing the non-denaturing polyacrylamide gel, the axl-miR-214sponge conjugate appeared to be stable for 8 hours, after which it began to degrade.

Subsequently, to assess the delivery of the miR-214 sponge to tumor cells expressing *axl*, breast tumor (4175-TGL 214) or melanoma (MA-2 214) cells overexpressing miR-214 were treated with the axl aptamer alone, the axl-miR-214sponge chimeric complex, an unrelated/scramble chimeric complex (scr-miR-214sponge), or were left untreated (ctrl). RNA was isolated 48 hours after treatments and miR-214 levels were measured by qRT-PCR. Alternatively, the cells were transfected with an anti-miR-214 (anti-214) or with the control sequence (anti-ctrl). A significant reduction in miR-214 levels was observed in the *axl*-expressing 4175-TGL 214 (Figure 1e) or MA-2 214 (Figure 1f) cells following treatment with the axl-miR-214sponge complex compared to the other treatments, and this modulation also occurred in the anti-214 transfected cells. Importantly, when *axl*-negative SKBR3 breast tumor cells were treated with the chimeric complex, no modulation of miR-214 was detected (Figure 1g), but when the same cells were transfected with anti-214, miR-214 expression appeared to be reduced compared to the control. This indicates that the aptamer axl works selectively in delivering the miR-214 sponge into cells expressing *axl.* Even when miR-214 levels in SKBR3 cells were increased by transfection with a miR-214 precursor (pre-214) and the cells were treated as indicated above, no changes in miR-214 levels were observed (Figure 1h).

### The axl-miR-214sponge complex reduces the motility of breast tumor and melanoma cells but has no effect on in vitro proliferation.

The inventors assessed whether axl-miR-214sponge is capable of acting on tumor spread, as miR-214 is a pro-metastatic miRNA (Penna, E, Orso, F et al. (2011). "microRNA-214 contributes to melanoma tumour progression through suppression of TFAP2C". EMBO J 30: 1990-2007). Specifically, migration, invasiveness through Matrigel, and transendothelial migration through a HUVEC layer, simulating the extravasation process, were analyzed. To perform these experiments, the cells (4175-TGL 214, MA-2 214, and SKBR3) were either left untreated or were treated with the anti-*axl* aptamer alone (axl) or in combination with the miR-214 sponge (axl-miR-214sponge), with the sponge bound to a scramble aptamer (scr-miR-214sponge), or in parallel, the cells were transfected with anti-214 or its negative control (anti-ctrl).

Cell motility inhibition was observed in *axl*-positive 4175-TGL 214 breast tumor (Figure 2a,c,e) and MA-2 214 melanoma (Figure 2b, d, f) cells, but not in SKBR3 cells that do not express the *axl* receptor (Figure 2g) compared to controls (ctrl or scr-miR-214sponge). Aptamer axl alone also blocks the metastatic traits in *axl*-expressing cells, but the effect of the axl-miR-214sponge conjugate is enhanced due to the combination of the anti*-axl* aptamer and miR-214 sponge activities. Similar results were also observed in anti-214-transfected cells as compared to controls (anti-ctrl), in both cells expressing and not expressing the *axl* receptor. These data suggest that the biological effects obtained depend on the specific aptamer-mediated delivery and are specific to cells expressing *axl* on their surface. When proliferation was investigated in tumor cells, no significant change in treated or transfected cells was observed (Figure 3a-f), confirming that miR-214 only affects cell movement. These data indicate the possibility of using the complex to block tumor cell motility and subsequent metastasization.

### The axl-miR-214sponge chimeric complex modulates the levels of both direct and indirect miR-214 targets in axl-expressing breast tumor and melanoma cells.

Since miRNA function is exerted by the negative regulation of their target genes, TFAP2γ and ITGα3, i.e., two direct miR-214 targets, were analyzed (Penna, E, Orso, F et al. (2011). "microRNA-214 contributes to melanoma tumour progression through suppression of TFAP2C". EMBO J 30: 1990-2007). It was therefore investigated whether miR-214sponge could act on these two molecules when 4175-TGL 214, MA-2 214 or SKBR3 cells were treated with the anti*-axl* aptamer alone, with axl-miR-214sponge, with scr-miR-214sponge, or left untreated (ctrl) or, alternatively, transfected with anti-214 or anti-ctrl. As shown, axl-miR-214sponge increases TFAP2γ and ITGα3 protein expression compared to ctrl, anti*-axl* or scr-miR-214sponge in *axl*-expressing cells (Figure 4a-b), whereas no effect occurred when SKBR3 cells were treated under the same conditions (Figure 4c). In addition, all cell lines showed a reduction in target genes when transfected with anti-214 compared to anti-ctrl. In parallel, miR-214 coordinates a pathway that includes not only adhesion molecules (ITGα3) and transcription factors (TFAP2γ), but also miR-148b anti-metastatic miRNA and its direct targets such as ALCAM and ITGα5 (Penna, E, et al. (2013) "miR-214 coordinates melanoma progression by upregulating ALCAM through TFAP2 and miR-148b downmodulation", Cancer Res 73: 4098-4111). axl-miR-214sponge is also able to act on indirect miR-214 targets, so when *axl*-positive cells were treated with the chimeric complex, a decrease in ALCAM protein levels was found (Figure 4d-e), whereas no effect was detected in SKBR3 cells (Figure 4f). These data indicate that axl-miR-214sponge works not only on direct miR-214 targets but also on the axis it forms with miR-148b.

### The axl-miR-214sponge chimeric complex reduces the number and size of the mammospheres

A mammosphere assay was performed to test whether the axl-miR-214sponge conjugate can act on a 3D model. Specifically, single cells from the 4175-TGL 214 or the SKBR3 line were plated (Day 0) and treated on Days 0, 3 and 5 with aptamers axl, axl-miR-214sponge or scr-miR-214sponge, or were left untreated (ctrl), and the mammospheres were analyzed on Day 5 (Figure 5a). When mammospheres derived from *axl*-expressing 4175-TGL 214 cells were examined on Day 5, significant reduction in the number (Figure 5b) and size (Figure 5c) thereof was observed in cells treated with axl-miR-214sponge compared to those treated with ctrl, axl or scr-miR-214sponge. In addition, when the above treatments were used on mammospheres derived from *axl*-negative SKBR3 cells, no change in number or size was detected (Figure 5d-e). These data indicate that axl-miR-214sponge controls both formation and growth of the mammospheres and is able to penetrate even within 3D structures, suggesting the possibility that the chimeric complex may be able to enter the tumor mass to perform its anti-tumor function.

### The axl-miR-214sponge chimeric complex blocks tumor cell spread

To assess the efficacy of the axl-miR-214sponge chimeric complex on metastatic spread in mice, RFP-expressing 4175-TGL breast tumor cells pre-treated for 24 hours with either the anti-*axl* aptamer or axl-miR-214sponge were injected into the mammary gland of NOD/SCID/IL2R_null (NSG) immunocompromised mice, and axl or axl-miR-214sponge was injected into the tumor mass 3 times a week starting on day 2 (Figure 6a). Metastasis formation was analyzed 23 days after injection of the tumor cells, and a decrease in the number of lung metastases was observed (Figure 6b) in mice treated with axl-miR-214sponge compared to those treated with axl. H&E tests on the primary mass showed an increase in necrosis (Figure 6c), while immunohistochemistry tests on cleaved caspase-3 and KI67 showed an increase in apoptosis (Figure 6d), but no changes in proliferation (Figure 6e) in tumors treated with the chimeric complex compared to those injected with axl alone. Importantly, no toxicity was observed following the administration of the aptamer alone or conjugated with miR-214 sponge. In fact, no morphological or weight changes occur in the organs analyzed, i.e., the liver, spleen, and kidneys (Figure 7a-c). In another experiment, 4175-TGL cells were injected, and the mice were injected into the tail vein from day 3 with PBS or axl-miR-214spongePEG (Figure 8a), i.e., a complex that differs from axl-miR-214sponge in the addition of a PEG molecule at the 5' that is intended to increase the weight of the chimeric complex and, consequently, reduce its renal filtration, thereby increasing its persistence in the circulation. Mice injected with the complex showed a reduction in lung metastases compared to mice injected with PBS, indicating that systemic administration of the compound is possible (Figure 8b).

## Claims

1. An isolated chimeric complex comprising:
a) an anti*-axl* aptamer comprising, from the 5' end to the 3' end:
(i) a single stranded nucleic acid sequence capable of binding to the tyrosine kinase receptor *axl*;
(ii) a linker element consisting of a linear unsubstituted alkyl chain containing 4 to 20 carbon atoms; and
(iii) a first sticky sequence consisting of a single stranded nucleic acid sequence 15 to 20 bases in length; and
b) an oligonucleotide containing a DNA sponge directed against miR-214, the oligonucleotide comprising from the 5' end to the 3' end:
(iv) a binding sequence consisting of a single stranded DNA sequence containing a plurality of miR-214 binding regions interspersed with one or more linking regions, and
(v) a second sticky sequence consisting of a single stranded DNA sequence of the same length in bases as the first sticky sequence identified in (iii) and complementary thereto.

2. The isolated chimeric complex according to claim 1, wherein the anti*-axl* aptamer is an RNA aptamer or a DNA aptamer.

3. The isolated chimeric complex according to claim 1, wherein the single stranded nucleic acid sequence capable of binding to the tyrosine kinase receptor *axl* identified in (i) is SEQ ID NO:1 or the DNA sequence corresponding thereto wherein each U (uridine) of SEQ ID NO: 1 is replaced by a dU (deoxyuridine).

4. The isolated chimeric complex according to any one of claims 1 to 3, wherein the first sticky sequence identified in (iii) is SEQ ID NO:2 or the DNA sequence corresponding thereto wherein each U (uridine) of SEQ ID NO:2 is replaced by a dU (deoxyuridine).

5. The isolated chimeric complex according to any one of claims 1 to 4, wherein the second sticky sequence identified in (v) is SEQ ID NO:4.

6. The isolated chimeric complex according to any one of claims 1 to 5, wherein the linear unsubstituted alkyl chain identified in (ii) contains 6 to 18 carbon atoms.

7. The isolated chimeric complex according to any one of claims 1 to 6, wherein the binding sequence identified in (iv) is selected from the group consisting of SEQ ID NO:3, SEQ ID NO:7, SEQ ID NO:8, SEQ ID NO:9, SEQ ID NO:10, SEQ ID NO:11, SEQ ID NO:12, SEQ ID NO:13, SEQ ID NO:14, SEQ ID NO:15, SEQ ID NO:16, SEQ ID NO:17, SEQ ID NO:18, SEQ ID NO:19, SEQ ID NO:20, SEQ ID NO:21, SEQ ID NO:22, SEQ ID NO:23, SEQ ID NO:24, SEQ ID NO:25, SEQ ID NO:26, SEQ ID NO:27, and SEQ ID NO:28.

8. The isolated chimeric complex according to any one of claims 1 to 7, wherein one or more pyrimidine bases are replaced by the corresponding 2'-fluoropyrimidine.

9. The isolated chimeric complex according to any one of claims 1 to 8, wherein one or more purine bases are replaced by the corresponding 2'-O-methylpurine.

10. The isolated chimeric complex according to any one of claims 1 to 9, for use in the therapeutic treatment of a tumor expressing the tyrosine kinase receptor *axl.*

11. The isolated chimeric complex for use according to claim 10, wherein the tumor is **characterized by** overexpression of the tyrosine kinase receptor *axl* and of miR-214.

12. The isolated chimeric complex for use according to claim 10, wherein the tumor is selected from the group consisting of melanoma, pancreatic tumor, stomach tumor, prostate tumor, lung tumor, breast tumor, ovary tumor.

13. The isolated chimeric complex for use according to any one of claims 10 to 12, wherein the treatment inhibits or reduces the onset and/or progression of metastases.

14. A pharmaceutical composition comprising an isolated chimeric complex according to any one of claims 1 to 9, and at least one pharmaceutically acceptable vehicle, excipient and/or diluent.

15. The pharmaceutical composition according to claim 14, which is in a pharmaceutical form suitable for subcutaneous, intravenous, intra-arterial, intraperitoneal, intramuscular, intranasal, inhalatory or intra-tumor administration.

16. The pharmaceutical composition according to claim 14 or 15, for use in the therapeutic treatment of a tumor expressing the tyrosine kinase receptor *axl.*

17. The pharmaceutical composition for use according to claim 16, wherein the tumor is **characterized by** the overexpression of the tyrosine kinase receptor *axl* and of miR-214.

18. The pharmaceutical composition for use according to claim 16, wherein the tumor is selected from the group consisting of melanoma, pancreatic tumor, stomach tumor, prostate tumor, lung tumor, breast tumor, ovary tumor.

19. The pharmaceutical composition for use according to any one of claims 16 to 18, wherein the treatment inhibits or reduces the onset and/or progression of metastases.

## Patentansprüche

1. Isolierter chimärer Komplex umfassend:
a) ein Anti-*axl*-Aptamer umfassend, von dem 5'-Ende zu dem 3'-Ende:
(i) eine einzelsträngige Nukleinsäuresequenz, die in der Lage ist, an den Rezeptor-Tyrosinkinase *axl* zu binden;
(ii) ein Verknüpfungselement bestehend aus einer linearen unsubstituierten Alkylkette enthaltend 4 bis 20 Kohlenstoffatomen;
(iii) eine erste kohäsive Sequenz, bestehend aus einer einzelsträngigen Nukleinsäuresequenz von 15 bis 20 Basen in Länge; und
b) ein Oligonukleotid, das einen DNA-Schwamm, der gegen miR-214 gerichtet wird, enthält, wobei das Oligonukleotid von dem 5'-Ende zu dem 3'-Ende umfasst:
(iv) eine Bindungssequenz bestehend aus einer einzelsträngigen DNA-Sequenz, die eine Mehrzahl von miR-214-Bindungsregionen enthält, die sich mit einer oder mehreren Verknüpfungsregionen abwechseln, und
(v) eine zweite kohäsive Sequenz bestehend aus einer einzelsträngigen DNA-Sequenz mit derselben Basenlänge wie die erste kohäsive Sequenz, die in (iii) identifiziert wird und komplementär dazu ist.

2. Isolierter chimärer Komplex nach Anspruch 1, wobei das Anti-*axl*-Aptamer ein RNA-Aptamer oder ein DNA-Aptamer ist.

3. Isolierter chimärer Komplex nach Anspruch 1, wobei die einzelsträngige Nukleinsäuresequenz, die in der Lage ist, an den Rezeptor-Tyrosinkinase-Rezeptor *axl* zu binden, SEQ ID NO: 1 ist oder die entsprechende DNA-Sequenz, wobei jedes U (Uridin) der SEQ ID NO: 1 durch ein dU (Deoxyuridin) ersetzt wird.

4. Isolierter chimärer Komplex nach einem der Ansprüche 1 bis 3, wobei die erste kohäsive Sequenz, die in (iii) identifiziert wird, SEQ ID NO: 2 ist oder die entsprechende DNA-Sequenz, wobei jedes U (Uridin) der SEQ ID NO: 2 durch ein dU (Deoxyuridin) ersetzt wird.

5. Isolierter chimärer Komplex nach einem der Ansprüche 1 bis 4, wobei die zweite kohäsive Sequenz, die in (v) identifiziert wird, SEQ ID NO: 4 ist.

6. Isolierter chimärer Komplex nach einem der Ansprüche 1 bis 5, wobei die lineare unsubstituierte Alkylkette, die in (ii) identifiziert wird, 6 bis 18 Kohlenstoffatome enthält.

7. Isolierter chimärer Komplex nach einem der Ansprüche 1 bis 6, wobei die Bindungssequenz, die in (iv) identifiziert wird, aus der Gruppe bestehend aus SEQ ID NO: 3, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27 und SEQ ID NO: 28 ausgewählt wird.

8. Isolierter chimärer Komplex nach einem der Ansprüche 1 bis 7, wobei eine oder mehrere Pyrimidinbasen durch die entsprechende 2'-Fluorpyrimidinbase ersetzt werden.

9. Isolierter chimärer Komplex nach einem der Ansprüche 1 bis 8, wobei eine oder mehrere Purinbasen durch die entsprechende 2'-O-Methylpurine ersetzt werden.

10. Isolierter chimärer Komplex nach einem der Ansprüche 1 bis 9, zur Verwendung bei der therapeutischen Behandlung eines Tumors, der den Rezeptor-Tyrosinkinase *axl* exprimiert.

11. Isolierter chimärer Komplex zur Verwendung nach Anspruch 10, wobei der Tumor durch eine Überexpression des Rezeptor-Tyrosinkinase *axl* und von miR-214 gekennzeichnet ist.

12. Isolierter chimärer Komplex zur Verwendung nach Anspruch 10, wobei der Tumor aus der Gruppe bestehend aus Melanom, Pankreastumor, Magentumor, Prostatatumor, Lungentumor, Brusttumor und Ovarialtumor ausgewählt wird.

13. Isolierter chimärer Komplex zur Verwendung nach einem der Ansprüche 10 bis 12, wobei die Behandlung das Auftreten und/oder das Fortschreiten von Metastasen hemmt oder reduziert.

14. Pharmazeutische Zusammensetzung umfassend einen isolierten chimären Komplex nach einem der Ansprüche 1 bis 9 sowie mindestens ein pharmazeutisch akzeptables Trägermittel, einen Hilfsstoff und/oder ein Verdünnungsmittel.

15. Pharmazeutische Zusammensetzung nach Anspruch 14, die in einer pharmazeutischen Form vorhanden ist, die für eine subkutane, intravenöse, intraarterielle, intraperitoneale, intramuskuläre, intranasale, inhalative oder intratumorale Verabreichung geeignet ist.

16. Pharmazeutische Zusammensetzung nach Anspruch 14 oder 15 zur Verwendung bei der therapeutischen Behandlung eines Tumors, der den Rezeptor-Tyrosinkinase *axl* exprimiert.

17. Pharmazeutische Zusammensetzung zur Verwendung nach Anspruch 16, wobei der Tumor durch die Überexpression des Rezeptor-Tyrosinkinase *axl* und von miR-214 gekennzeichnet ist.

18. Pharmazeutische Zusammensetzung zur Verwendung nach Anspruch 16, wobei der Tumor aus der Gruppe bestehend aus Melanom, Pankreastumor, Magentumor, Prostatatumor, Lungentumor, Brusttumor und Ovarialtumor ausgewählt wird.

19. Pharmazeutische Zusammensetzung zur Verwendung nach einem der Ansprüche 16 bis 18, wobei die Behandlung das Auftreten und/oder das Fortschreiten von Metastasen hemmt oder reduziert.

## Revendications

1. Complexe chimérique isolé comprenant :
a) un aptamère anti*-axl* comprenant, de l'extrémité 5' à l'extrémité 3' :
(i) une séquence d'acide nucléique simple brin capable de se lier au récepteur tyrosine kinase *axl* ;
(ii) un élément de liaison consistant en une chaîne alkyle linéaire non substituée contenant de 4 à 20 atomes de carbone ;
(iii) une première séquence cohésive consistant en une séquence d'acide nucléique simple brin de 15 à 20 bases de longueur ; et
b) un oligonucléotide contenant une éponge d'ADN dirigée contre miR-214, l'oligonucléotide comprenant, de l'extrémité 5' à l'extrémité 3' :
(iv) une séquence de liaison consistant en une séquence d'ADN simple brin contenant une pluralité de régions de liaison à miR-214 entrecoupées d'une ou de plusieurs régions de liaison, et
(v) une seconde séquence cohésive consistant en une séquence d'ADN simple brin ayant la même longueur en bases que la première séquence cohésive identifiée en (iii) et complémentaire de celle-ci.

2. Complexe chimérique isolé selon la revendication 1, dans lequel l'aptamère anti-*axl* est un aptamère ARN ou un aptamère ADN.

3. Complexe chimérique isolé selon la revendication 1, dans lequel la séquence d'acide nucléique simple brin capable de se lier au récepteur tyrosine kinase *axl* identifiée en (i) est la SEQ ID NO: 1 ou la séquence d'ADN correspondante dans laquelle chaque U (uridine) de la SEQ ID NO: 1 est remplacé par un dU (désoxyuridine).

4. Complexe chimérique isolé selon l'une quelconque des revendications 1 à 3, dans lequel la première séquence cohésive identifiée en (iii) est la SEQ ID NO: 2 ou la séquence d'ADN correspondante dans laquelle chaque U (uridine) de la SEQ ID NO: 2 est remplacé par un dU (désoxyuridine).

5. Complexe chimérique isolé selon l'une quelconque des revendications 1 à 4, dans lequel la seconde séquence cohésive identifiée en (v) est la SEQ ID NO: 4.

6. Complexe chimérique isolé selon l'une quelconque des revendications 1 à 5, dans lequel la chaîne alkyle linéaire non substituée identifiée en (ii) contient de 6 à 18 atomes de carbone.

7. Complexe chimérique isolé selon l'une quelconque des revendications 1 à 6, dans lequel la séquence de liaison identifiée en (iv) est choisie dans le groupe consistant en SEQ ID NO: 3, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27 et SEQ ID NO: 28.

8. Complexe chimérique isolé selon l'une quelconque des revendications 1 à 7, dans lequel une ou plusieurs bases pyrimidiques sont remplacées par la 2'-fluoropyrimidine correspondante.

9. Complexe chimérique isolé selon l'une quelconque des revendications 1 à 8, dans lequel une ou plusieurs bases puriques sont remplacées par la 2'-O-méthylpurine correspondante.

10. Complexe chimérique isolé selon l'une quelconque des revendications 1 à 9, destiné à être utilisé dans le traitement thérapeutique d'une tumeur exprimant le récepteur tyrosine kinase *axl.*

11. Complexe chimérique isolé pour utilisation selon la revendication 10, dans lequel la tumeur est **caractérisée par** une surexpression du récepteur tyrosine kinase *axl* et de miR-214.

12. Complexe chimérique isolé pour utilisation selon la revendication 10, dans lequel la tumeur est choisie dans le groupe consistant en un mélanome, une tumeur pancréatique, une tumeur de l'estomac, une tumeur de la prostate, une tumeur du poumon, une tumeur du sein, une tumeur de l'ovaire.

13. Complexe chimérique isolé pour utilisation selon l'une quelconque des revendications 10 à 12, dans lequel le traitement inhibe ou réduit l'apparition et/ou la progression des métastases.

14. Composition pharmaceutique comprenant un complexe chimérique isolé selon l'une quelconque des revendications 1 à 9, et au moins un véhicule, excipient et/ou diluant pharmaceutiquement acceptable.

15. Composition pharmaceutique selon la revendication 14, qui est sous une forme pharmaceutique adaptée à une administration sous-cutanée, intraveineuse, intra-artérielle, intrapéritonéale, intramusculaire, intranasale, inhalatoire ou intra-tumorale.

16. Composition pharmaceutique selon la revendication 14 ou 15, destinée à être utilisée dans le traitement thérapeutique d'une tumeur exprimant le récepteur tyrosine kinase axl.

17. Composition pharmaceutique pour utilisation selon la revendication 16, dans laquelle la tumeur est **caractérisée par** la surexpression du récepteur tyrosine kinase *axl* et de miR-214.

18. Composition pharmaceutique pour utilisation selon la revendication 16, dans laquelle la tumeur est choisie dans le groupe consistant en un mélanome, une tumeur pancréatique, une tumeur de l'estomac, une tumeur de la prostate, une tumeur du poumon, une tumeur du sein, une tumeur de l'ovaire.

19. Composition pharmaceutique pour utilisation selon l'une quelconque des revendications 16 à 18, dans laquelle le traitement inhibe ou réduit l'apparition et/ou la progression des métastases.
